(19) Europäisches Patentamt
European Patent Office
Office européen des brevets

(11) Publication number: 0 126 252 A1

(12) EUROPEAN PATENT APPLICATION

(21) Application number: 84103745.0

(22) Date of filing: 05.04.84

(51) Int. Cl.³: C 07 D 209/48, C 07 D 307/89

(30) Priority: 20.04.83 US 486618

(43) Date of publication of application: 28.11.84 Bulletin 84/48

(84) Designated Contracting States: DE FR GB IT NL

(71) Applicant: GENERAL ELECTRIC COMPANY, 1 River Road, Schenectady New York 12305 (US)

(72) Inventor: Evans, Thomas Lane, 70 Randi Road, Schenectady New York 12309 (US)

(74) Representative: Catherine, Alain et al, GETSCO 42, avenue Montaigne, F-75008 Paris (FR)

(54) **Sulfur-containing aromatic compositions and methods for their preparation and use.**

(57) New alkali metal mercaptides and disulfides have the formula $Z^1$–S–$Z^2$, wherein $Z^1$ is an alkali metal or –S–$Z^2$ and $Z^2$ is a phthalic acid derivative moiety, typically a phthalic anhydride or phthalimide moiety. The mercaptides ($Z^1$ is an alkali metal) may be prepared by reacting an alkali metal sulfide with a substituted phthalic acid derivative in a molar ratio of at least about 1 : 1. The disulfides ($Z^1$ is –S–$Z^2$) may be prepared by mild oxidation of the corresponding alkali metal mercaptides. The alkali metal mercaptides are useful as intermediates in the preparation of thioether phthalic acid derivatives. The disulfides are useful in the preparation of polyimides by reaction with diamines.

EP 0 126 252 A1

# SULFUR-CONTAINING AROMATIC COMPOSITIONS AND METHODS FOR THEIR PREPARATION AND USE

This invention relates to new sulfur-containing aromatic compositions of matter and methods for their preparation and use. In their broadest definition, the compositions of this invention have the formula $Z^1$-S-$Z^2$, wherein $Z^1$ is an alkali metal or -X-$Z^2$ and $Z^2$ is a phthalic acid derivative moiety.

The use of aromatic bisimides and dianhydrides in the preparation of polyimides and intermediates therefor is known in the art. Various sulfur-containing compounds of this type can be so used. Reference is made, for example, to U.S. Patents 4,102,905 and 4,107,147, the disclosures of which are incorporated by reference herein. The first of these describes the preparation of bis(sulfone) aromatic dianhydrides by the reaction of an aromatic dithiol with a substituted phthalic anhydride followed by oxidation of the sulfur atoms to sulfone groups. (Analogous dianhydrides may be prepared by a similar reaction starting with an aliphatic dithiol.) The second patent describes the preparation of polysulfoneimides by the reaction of the bis(sulfone) aromatic dianhydrides with diamines. Very few dithiols, however, are commercially available at a price low enough for economical use in the preparation of such sulfur-containing compounds.

A principal object of the present invention, therefore, is to prepare chemical intermediates which may be efficiently and inexpensively converted into sulfur-containing phthalic acid derivatives, such as dianhydrides and bisimides.

A further object is to provide a relatively easy and inexpensive route for such dianhydrides, bisimides and the like.

Another object is to prepare certain novel disulfides and polyimides derived therefrom.

Other objects will in part be obvious and will in part appear hereinafter.

As will be apparent from the definition of $Z^1$, the novel sulfur-containing aromatic compounds of this invention are alkali metal mercaptides and disulfides. The alkali metal in the mercaptide may be, for example, lithium, sodium or potassium; sodium is preferred because of its availability and relatively low cost.

The $Z^2$ moiety in the above formula is a phthalic acid derivative moiety. By "phthalic acid derivative" is meant the free acid and the anhydride, salts, esters (including acidic esters), amides (including acid amides) and imides thereof. The $Z^2$ moiety is most often a phthalic anhydride or phthalimide moiety having the formula

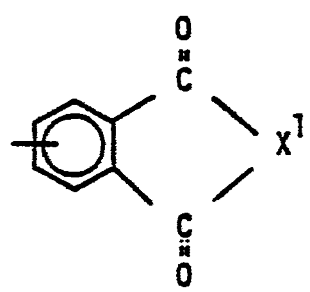

,

wherein $X^1$ is O or N-$R^1$ and $R^1$ is hydrogen, an alkyl radical having 1-8 carbon atoms or an aromatic radical having 6-20 carbon atoms.

Because of ease of preparation and reaction, the preferred compounds are those in which $X^1$ is N-$R^1$. Most preferred are those in which the sulfur atom is attached in the 4-position.

The $R^1$ radical may be, for example, hydrogen, methyl, ethyl, n-propyl, isopropyl, n-butyl, n-hexyl, n-octyl, 2-ethylhexyl, phenyl, tolyl, xylyl, 1-naphthyl, 2-naphthyl or methoxyphenyl. When $R^1$ is alkyl, it ordinarily contains no more than about 4 carbon atoms; when it is aryl, it is normally phenyl or substituted phenyl. Alkyl radicals are preferred, especially methyl.

The alkali metal mercaptides of this invention may be prepared by reacting an alkali metal sulfide with a substituted phthalic acid derivative of the formula $X^2-Z^2$, wherein $X^2$ is halo, nitro or $-S-Z^2$, the molar ratio of said alkali metal sulfide to said substituted phthalic acid derivative being at least about 1:1. The preferred alkali metal sulfide is sodium sulfide ($Na_2S$). The substituent $X^2$ is preferably chloro, for reasons of ease of preparation, relatively low cost and capability of clean conversion into the compounds of this invention in high yield.

The reaction between the alkali metal sulfide and the substituted phthalic acid derivative is typically effected by merely mixing the two reagents and heating the mixture at a temperature within the range of about 100-200°C, preferably about 120-175°C, until reaction is complete. Because the alkali metal mercaptide is easily oxidized (as described hereinafter) upon contact with oxygen or air, it is necessary to carry out the reaction and store the product in an inert atmosphere; i.e., under nitrogen, helium or the like. The use of a substantially inert diluent for the reaction is preferred. Illustrative diluents are aromatic hydrocarbons such as toluene, xylene, chlorinated aromatic hydrocarbons such as chlorobenzene and o-dichlorobenzene, and dipolar aprotic solvents such dimethylformamide, dimethylacetamide, dimethyl sulfoxide and N-methylpyrrolidone. The dipolar aprotic solvents, especially dimethylformamide, are preferred.

In order to produce the alkali metal mercaptide, it is necessary that the molar ratio of alkali metal sulfide to substituted phthalic acid derivative be at least about 1:1. The reaction of an alkali metal sulfide with a substituted phthalimide in a molar ratio of 1:2 under similar conditions to yield the corresponding bis-phthalimide sulfide is described in U.S. Patent 4,054,584, the disclosure of which is incorporated by reference herein. As previously noted, however, this sulfide may itself be converted to the alkali metal mercaptide by further reaction with

alkali metal sulfide, provided at least one mole of the latter is used per mole of bis-phthalimide sulfide. The same is true of sulfides of the other phthalic acid derivatives. Thus, the use of substantially less than a 1:1 molar ratio of alkali metal sulfide to substituted phthalic acid derivative in the preparation of the mercaptide can easily be corrected by merely increasing the proportion of alkali metal sulfide in the reaction mixture.

For the preparation of the alkali metal mercaptide, there does not appear to be any upper limit of the molar ratio of alkali metal sulfide to substituted phthalic acid derivative. However, it is necessary to remove excess alkali metal sulfide before further reaction with an electrophilic compound; otherwise the sulfide will compete with the mercaptide in said further reaction. For this and other reasons, there is seldom any purpose in employing a higher molar ratio than about 1.2:1, and an upper limit of about 1.05:1 is usually preferred.

The alkali metal mercaptides of this invention may be isolated by conventional means such as evaporation of the solvent or precipitation by addition of a non-solvent therefor, provided an inert atmosphere is maintained. However, isolation is seldom necessary since the principal utility of the mercaptides is as chemical intermediates. Their conversion to other useful compounds may often be conveniently effected in the medium in which they were prepared by the addition of further reagents thereto. In the descriptions hereinafter of further reactions of the alkali metal mercaptides, it is assumed that they are used without isolation; however, it should be understood that they may be isolated if desired.

The disulfides of this invention (i.e., the compounds in which $Z^1$ is $-S-Z^2$) may be easily prepared by mild oxidation (e.g., with oxygen or air) of the corresponding alkali metal mercaptide. Such oxidation is often preferably effected in situ by exposing the mercaptide reaction mixture to oxygen or air during preparation. Isolation of the disulfide may be effected

by conventional methods such as those previously described.

The preparation of the compounds of this invention is illustrated by the following examples.

EXAMPLE 1

A solution of 0.5 gram (0.00256 mole) of 4-chloro-N-methylphthalimide, 0.2 gram (0.00256 mole) of sodium sulfide and 0.2 gram (0.0013 mole) of biphenyl (used as an internal standard) in 20 ml. of dimethylformamide was heated at 125°C, with stirring, in a nitrogen atmosphere, and was periodically analyzed by high pressure liquid-liquid chromatography. After 1 hour, the analysis showed the presence of a minor proportion of 4,4'-bis(N-methylphthalimide) sulfide and less than 1% 4-chloro-N-methylphthalimide. The major product was the desired sodium 4-(N-methylphthalimide) sulfide.

EXAMPLE 2

A solution of 0.4 gram (0.0011 mole) of 4,4'-bis(N-methylphthalimide) sulfide, 0.1 gram (0.0013 mole) of sodium sulfide and 0.19 gram (0.0012 mole) of biphenyl in 35 ml. of dimethylformamide was heated at 150°C under nitrogen, with sitrring, for 22 hours. Analysis by high pressure liquid-liquid chromatography showed that less than 3% of the 4,4'-bis(N-methylphthalimide) sulfide remained. The presence of a substantial proportion of sodium 4-(N-methylphthalimide) sulfide was shown by adding 0.43 gram (0.0022 mole) of 4-chloro-N-methylphthalimide and heating under nitrogen for 3 hours at 150°C, with stirring. Further analysis then showed a 96% yield of 4,4'-bis(N-methylphthalimide) sulfide, formed by the reaction of the chlorophthalimide with the sodium phthalimide sulfide.

EXAMPLE 3

A solution of 1.0 gram (0.0051 mole) of 4-chloro-N-methylphthalimide and 0.41 gram (0.00526 mole) of sodium sulfide in 30 ml. of dimethylformamide was heated under reflux for 24 hours in contact with air and was then cooled, acidified with glacial acetic acid, stirred for 2 hours and poured into water.

The solid product thus obtained was collected by filtration and was identified by high pressure liquid-liquid chromatography and mass spectrometry as the desired 4,4'-bis(N-methylphthalimide) disulfide. The yield was 50% of theoretical.

The alkali metal mercaptides of this invention are useful as chemical intermediates for the preparation of thioether phthalic acid derivatives. Such derivatives generally have the formula $R^2(SZ^2)_m$, wherein $R^2$ is an organic radical and m is from 1 to 3, and may be prepared by reaction of the mercaptide with at least one halide of the formula $R^2(X^3)_m$, wherein $X^3$ is halogen. This method for preparing thioether phthalic acid derivatives is one aspect of the present invention.

The organic radical comprising $R^2$ may be aliphatic (e.g., methyl, ethyl, propyl, butyl, hexyl, ethylene, propylene, trimethylene) or alicyclic (e.g., cyclopentyl, cyclohexyl, cyclohexylene) and is then usually a hydrocarbon radical, preferably aliphatic and containing about 2-4 carbon atoms; or it may be aromatic (e.g., phenyl, tolyl, naphthyl, methoxyphenyl, cyanophenyl, biphenylyl, phenylene, tolylene, naphthylene, biphenylene, methylene-bis-phenyl) and then usually contains about 6-10 carbon atoms and is preferably a hydrocarbon radical. The $X^2$ value is usually chlorine or bromine, and m is most often 1 or 2. Illustrative halides which may be reacted with the alkali metal mercaptides are ethyl bromide, ethylene dibromide, chlorobenzene, p-dichlorobenzene, p-methoxychlorobenzene and p-chlorobenzonitrile.

The reaction producing the thioether phthalic acid derivative is typically conducted at temperatures in the range of about 100-200°C in an inert atmosphere. It involves approximately equivalent proportions of the two reagents; e.g., a ratio of equivalents of halide to mercaptide of about 0.9-1.1:1. (For the purpose of this invention, the equivalent weight of a halide is its molecular weight divided by the number of replaceable halogen atoms therein. The equivalent weights of the mercaptides of this invention are, of course, equal to their molecular weights.) The reaction typically involves the use of a diluent, most often the same one used in the preparation of the mercaptide.

The preparation of thioether phthalic acid derivatives by the method of this invention is illustrated by the following examples.

EXAMPLE 4

A mixture of one gram (0.0051 mole) of 4-chloro-N-methylphthalimide sulfide, 0.41 gram (0.00526 mole) of sodium sulfide and 30 ml. of dimethylformamide was heated under reflux for 24 hours to produce sodium 4-(N-methylphthalimide) sulfide. To the solution thereof was added 3 grams (0.28 mole) of ethyl bromide. Refluxing was continued for one hour, after which the mixture was cooled and filtered and the solvent was removed by vacuum evaporation. There was obtained a 72% yield of the desired ethyl 4-(N-methylphthalimide) sulfide; its structure was confirmed by mass spectrometry.

EXAMPLE 5

Following the procedure of Example 4, 0.0051 mole each of 4-chloro-N-methylphthalimide and sodium sulfide were reacted in.dimethylformamide to produce sodium 4-(N-methylphthalimide) sulfide. To the solution thereof was added 0.69 gram (0.005 mole) of p-chlorobenzonitrile. The mixture was heated under reflux for 20 hours under nitrogen and the products were separated by liquid chromatography. There was obtained p-cyanophenyl 4-(N-methylphthalimide) sulfide having a melting point of 140°C.

EXAMPLE 6

Following the procedure of Example 4, 0.0051 mole each of 4-chloro-N-methylphthalimide and sodium sulfide were reacted in dimethylformamide to produce sodium 4-(N-methylphthalimide) sulfide. To the solution thereof was added 0.48 gram (0.0026 mole) of ethylene dibromide. The mixture was heated at 100°C for 1 hour with stirring and poured into water. The desired ethylene 1,2-bis(4-N-methylphthalimide) sulfide was separated by filtration and dried; it had a melting point of 175°C.

The novel disulfides of this invention and the difunctional thioether phthalic acid derivatives produced as described hereinabove are useful as intermediates for the preparation of polyimide resins. The difunctional thioether phthalic acid derivatives may also be oxidized to the corresponding sulfone phthalic acid derivatives by methods similar to those disclosed in the aforementioned U.S. Patent 4,102,905; the sulfone phthalic acid derivatives may likewise be used to produce polyimide resins.

The polyimides are conveniently prepared by reacting the dianhydrides, optionally in admixture with other known dianhydrides, with diamines. Suitable diamines include those of the formula $H_2N-Q-NH_2$, wherein Q is an aromatic hydrocarbon radical containing about 6-20 carbon atoms or a halogenated derivative thereof, an alkylene or cycloalkylene radical containing about 2-20 carbon atoms, or a bis-alkylenepoly(dialkylsiloxane) radical. The aromatic hydrocarbon radicals are preferred.

Examples of suitable diamines are ethylenediamine, propylenediamine, trimethylenediamine, diethylenetriamine, triethylenetetramine, heptamethylenediamine, octamethylenediamine, 2,11-dodecanediamine, 1,12-octadecanediamine, 3-methylheptamethylenediamine, 4,4-dimethylheptamethylenediamine, 4-methylnonamethylenediamine, 2,5-dimethylhexamethylenediamine, 2,2-dimethylpropylenediamine, N-methyl-bis(3-aminopropyl)amine, 3-methoxyhexamethylenediamine, 1,2-bis(3-aminopropoxy)ethane, bis(3-aminopropyl) sulfide, 1,4-cyclohexanediamine, bis-(4-aminocyclohexyl)methane, m-phenylenediamine, p-phenylenediamine, 2,4-diaminotoluene, 2,6-diaminotoluene, m-xylylenediamine, p-xylylenediamine, benzidine, 3,3'-dimethylbenzidine, 3,3'-dimethoxybenzidine, 1,5-diaminonaphthalene, 4,4'-diaminodiphenylmethane, 4,4'-diaminodiphenylpropane, 2,4-bis-(β-amino-t-butyl)toluene, bis(p-β-methyl-o-aminopentyl)benzene, 1,3-diamino-4-isopropylbenzene, 4,4-diaminodiphenyl sulfone, 4,4'-diaminodiphenyl ether and bis(3-aminopropyl) tetramethyldisiloxane. Mixtures of these diamines may also be used. Particularly preferred are the aromatic

diamines, especially m-phenylenediamine and 4,4'-diaminodiphenylmethane; the Q radical is then either

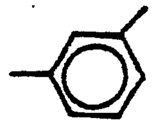 (preferably) or 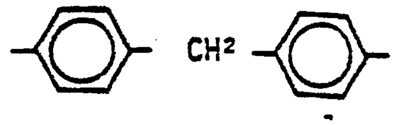 .

The polyimides may be prepared by merely heating a mixture of the dianhydride(s) and the diamine(s), typically with agitation and at a temperature from about 200° to about 280°C. In general, substantially equimolar amounts of diamine and dianhydride are used. The reaction is often conveniently effected by a melt polymerization procedure, although substantially inert diluents may be used if desired. Polymerization may be facilitated by purging with an inert gas such as nitrogen, and/or by reducing pressure to remove water of reaction. Molecular weight control may be effected by adding minor amounts of organic monoamines or organic monoanhydrides, including the monofunctional thioether phthalic anhydrides prepared as described hereinabove. For further description of the preparation and uses of such polyimides, reference is made to the aforementioned U.S. Patent 4,107,147.

Another aspect of the present invention comprises the polyimides derived from the novel disulfides. Such polyimides are characterized by units having the formula

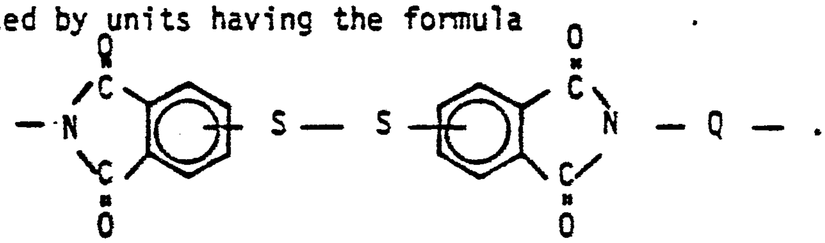

The preparation of the polyimides of this invention and similar polyimides derived from the thioether phthalic anhydrides is illustrated by the following examples.

## EXAMPLES 7-11

The phthalimides of Examples 3-6 are converted to the corresponding anhydrides by hydrolysis with excess 50% aqueous

sodium hydroxide, acidification, separation of the precipitated acid and conversion to the anhydride by treatment with acetic anhydride in a large excess of acetic acid, by a procedure similar to that described in Example 6 of the aforementioned U.S. Patent 4,054,584.

Mixtures of the anhydrides thus produced with diamines are heated at 250°C under nitrogen, with stirring, as water of reaction is removed by distillation. When water evolution ceases, the system is evacuated and the temperature is raised to 300°C. The desired mixtures are cooled and dissolved in m-cresol, and the polyimides are precipitated by pouring the m-cresol solution into methanol. The anhydrides and diamines used, and the molar proportions thereof, are given in the following table.

| | Dianhydride | | Monoanhydride | | Diamine | |
|---|---|---|---|---|---|---|
| Example | Example | Mole | Example | Mole | Q | Mole |
| 7 | 3 | 1.0 | -- | -- | m-$C_6H_4$ | 1.0 |
| 8 | 3 | 1.0 | -- | -- | p,p'-$C_6H_4CH_2C_6H_4$ | 1.0 |
| 9 | 6 | 1.0 | -- | -- | m-$C_6H_4$ | 1.0 |
| 10 | 3 | 0.98 | 4 | 0.04 | p,p'-$C_6H_4CH_2C_6H_4$ | 1.0 |
| 11 | 6 | 0.98 | 5 | 0.04 | m-$C_6H_4$ | 1.0 |

What is claimed is:

1. A sulfur-containing composition having the formula $Z^1$-S-Z² , wherein $Z^1$ is an alkali metal or -S-Z² and Z² is a phthalic acid derivative moiety.

2. A composition according to claim 1 which is an alkali metal mercaptide wherein $Z^1$ is an alkali metal.

3. A composition accoridng to claim 2 wherein Z² has the formula

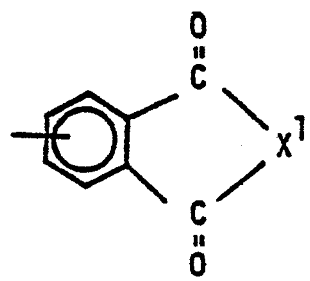

,

wherein $X^1$ is O or N-$R^1$ and $R^1$ is hydrogen, an alkyl radical having 1-8 carbon atoms or an aromatic radical having 6-20 carbon atoms.

4. A compound according to claim 3 wherein the sulfur atom is attached to the aromatic ring in the 4-position.

5. A compound according to claim 4 wherein $X^1$ is N-$R^1$.

6. A compound according to claim 5 wherein the alkali metal is sodium.

7. A compound according to claim 6 wherein $R^1$ is an alkyl radical having 1-4 carbon atoms.

8. A compound according to claim 7 wherein $R^1$ is methyl.

9. A compound according to claim 1 which is a disulfide wherein $Z^1$ is -S-Z².

10. A composition according to claim 9 wherein Z² has the formula

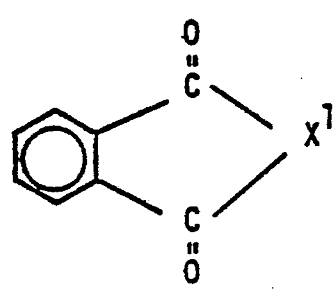

,

wherein $X^1$ is O or $N-R^1$ and $R^1$ is hydrogen, an alkyl radical having 1-8 carbon atoms or an aromatic radical having 6-20 carbon atoms.

11. A compound according to claim 10 wherein the sulfur atoms are attached to the aromatic rings in the 4-positions.

12. A compound using claim 11 wherein $X^1$ is $N-R^1$.

13. A compound according to claim 12 wherein $R^1$ is an alkyl radical having 1-4 carbon atoms.

14. A compound according to claim 13 wherein $R^1$ is methyl.

15. A polyimide characterized by units having the formula

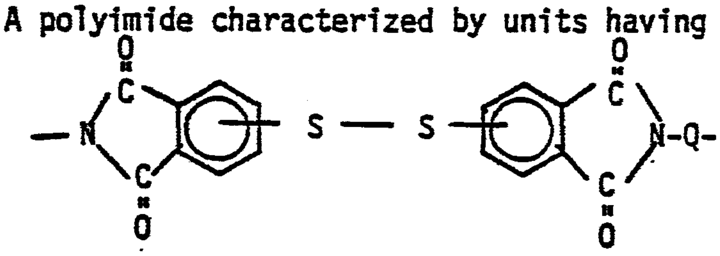 ,

wherein Q is derived from a diamine of the formula $H_2N-Q-NH_2$.

16. A polyimide according to claim 15 wherein the sulfur atoms are attached to the aromatic rings in the 4-positions.

17. A polyimide according to claim 16 wherein Q is an aromatic hydrocarbon radical containing about 6-20 carbon atoms or a halogenated derivative thereof, an alkylene or cycloalkylene radical containing about 2-20 carbon atoms, or a bis-alkylenepoly(dialkylsiloxane) radical.

18. A polyimide according to claim 17 wherein Q is an aromatic radical.

19. A polyimide according to claim 18 wherein Q is

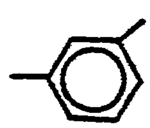 or 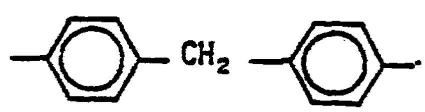 .

20. A method for preparing a compound according to any of claims 2-8 which comprises reacting, in an inert atmosphere, the corresponding alkali metal sulfide with the corresponding substituted phthalic acid derivative of the formula $X^2-Z^2$, wherein $X^2$ is halo, nitro, or $-S-Z^2$, the molar ratio of said alkali metal

sulfide to said substituted phthalic acid derivative being at least about 1:1.

21. A method for preparing a compound according to any of claims 9-14 which comprises mildly oxidizing the corresponding alkali metal mercaptide.

22. A method according to claim 21 wherein the oxidation is effected in situ by exposing the mercaptide reaction mixture to oxygen or air during preparation.

23. A method for preparing a thioether phthalic acid derivative of the formula $R^2(SZ^2)_m$, wherein $R^2$ is an organic radical and m is from 1 to 3, which comprises reacting the corresponding compound according to any of claims 2-8 with at least one halide of the formula $R^2(X^3)_m$, wherein $X^3$ is halogen.

24. A method according to claim 23 wherein $X^3$ is chlorine or bromine.

25. A method according to claim 24 wherein m is 1 or 2.

26. A method according to claim 25 wherein $R^2$ is an aliphatic hydrocarbon radical containing about 2-4 carbon atoms.

27. A method according to claim 25 wherein $R^2$ is an aromatic radical containing about 6-10 carbon atoms.

European Patent Office

# EUROPEAN SEARCH REPORT

0126252

Application number

EP 84 10 3745

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | GB-A-1 477 519 (GENERAL ELECTRIC CO.)<br>* Whole document *<br>--- | 1-27 | C 07 D 209/48<br>C 07 D 307/89 |
| D,A | US-A-4 054 584 (F.J. WILLIAMS III)<br>* Whole document *<br>--- | 1-27 | |
| A | US-A-3 922 284 (D.R. HEATH)<br>* Whole document *<br>--- | 1-27 | |
| A | US-A-3 933 862 (F.J. WILLIAMS III)<br>* Whole document *<br>--- | 1-27 | |
| D,A | US-A-4 102 905 (F.J. WILLIAMS III)<br>* Whole document *<br>--- | 1-27 | TECHNICAL FIELDS SEARCHED (Int. Cl. 3) |
| D,A | US-A-4 107 147 (F.J. WILLIAMS III)<br>* Whole document *<br>----- | 1-27 | C 07 D 209/00 |

The present search report has been drawn up for all claims

| Place of search | Date of completion of the search | Examiner |
|---|---|---|
| THE HAGUE | 10-08-1984 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document